# EUROPEAN PATENT SPECIFICATION

(11) **EP 1 680 097 B1**
(45) Date of publication and mention of the grant of the patent: **11.03.2009**
(21) Application number: 04796563.7
(22) Date of filing: 27.10.2004
(51) Int. Cl.: A61K 9/70

(54) **PREPARATIONS FOR TOPICAL APPLICATION AND METHODS OF DELIVERING AN ACTIVE AGENT TO A SUBSTRATE**
ZUBEREITUNGEN FÜR DIE TOPISCHE ANWENDUNG UND VERFAHREN ZUR ABGABE EINES WIRKSTOFFS AN EIN SUBSTRAT
PREPARATIONS POUR L'APPLICATION TOPIQUE ET METHODES D'APPORT D'UN AGENT ACTIF A UN SUBSTRAT

(30) Priority: 27.10.2003 US 514709 P
(43) Date of publication of application: 19.07.2006
(73) Proprietor: Dow Corning Corporation, Midland, MI 48686-0994 (US); GENENCOR INTERNATIONAL, INC., Palo Alto, California 94304-1013 (US)
(72) Inventor: BOTT, Richard, R., Burlingame, CA 94010 (US); BRANDSTADT, Kurt, Friedrich, Michigan 48642 (US); KOLLAR, Csilla, Midland, MI 48640 (US); LANE, Thomas, Howard, Midland, MI 48640 (US); LILES, Donald, Taylor, Midland, MI 48640 (US); SALDJENO, Mae, Daly City, CA 94015 (US); THOMAS, Xavier, Jean-Paul, F-09000 Foix (FR)
(74) Representative: Caldwell, Judith Margaret
(86) International application number: PCT/US2004/035686
(87) International publication number: WO 2005/044242

(56) References cited:
- WO-A-99/11247
- US-A1- 2003 180 281

## Description

### RELATED APPLICATIONS

The present application claims priority to United States Provisional Patent Application No. 60/514709, which was filed October 27, 2003.

### BACKGROUND OF THE INVENTION

### 1. Field of the Invention

The subject invention generally relates to a multi-layer dressing and a controlled-release composition for topical application to a substrate. The subject invention also generally relates to a method of delivering an active agent to the substrate. More specifically, the dressing, the controlled-release composition, and the method of this invention relate are used for topical application to skin and, more particularly, to controlled release dressings comprising emulsions of protein-containing active agents and silicone that, when applied to the skin for therapeutic purposes, provide controlled-release of the active agents from the dressing.

### 2. Description of the Related Art

Silicones are compounds based on alkylsiloxane or organosiloxane chemistry and include polydimethylsiloxane materials that have been used as excipients and process aids in pharmaceutical applications. Some of these materials have attained the status of pharmacopoeial compounds. Known in the art is the use of such silicone compounds in controlled transdermal drug delivery systems. New long lasting drug delivery applications including implant, insert, mucoadhesive, and transdermal forms draw on the unique and intrinsic properties of silicone. Transdermal delivery systems allow controlled-release of active molecules with biologically appropriate kinetics to a targeted area, and prevent the adverse effects, such as peak dosages, low compliance, and drug degradation, commonly observed with traditional oral and parenteral medication.

Transdermal drug delivery systems consist of drug containing adhesive patches, which adhere to intact skin up to 7 days. The patch design controls the release of the active agent, which is then transported through the skin and into the organism by the circulatory system for a systemic activity. For example, WO 99/11247 describes a biphasic lipid vesicle composition for transdermal administration of an immunogen for eliciting an immune response in a subject. The immunogen is suspended in an oil-in-water emulsion within a lipid vesicle and the immunogen is delivered through the lipid bilayer. Using the skin as an entry point, transdermal forms, which consist of an adhesive plaster or a film-forming and substantive material (e.g., cream or gel), are used for local treatment (muscle or skin disease). US 2003/0180281 describes a topical preparation comprising a water-in-oil emulsion in which an active agent in a hydrophilic carrier is suspended in a silicone matrix. However, transdermal drug delivery systems have not been incorporated into topical dressing applications such as wound dressings and ointments, wherein a biochemical agent dispersed within a silicone matrix is released onto skin or a wound to accelerate healing.

Accordingly, the need remains in the relevant art for preparations that take advantage of the beneficial properties of silicone, and can provide controlled release of active agents.

### SUMMARY OF THE INVENTION AND ADVANTAGES

A multi-layer dressing, a controlled-release composition, and a method are disclosed. These elements of the present invention are used for topical application to a substrate. The method delivers an active agent to the substrate.

The dressing includes a controlled-release layer and an adhesive layer. The controlled-release layer is formed from the controlled-release composition. The controlled-release composition, more specifically, includes an oil-in-water emulsion, and the active agent. The active agent is incorporated into the hydrophilic phase of the emulsion and comprises a protein. The hydrophilic phase comprises a silicone component. The adhesive layer is disposed adjacent the controlled-release layer for adhering, the dressing to the substrate. The method delivers the active agent comprising the protein to the substrate.

With the present invention, the active agent can be delivered to the substrate in a controlled manner upon application to the substrate.

### BRIEF DESCRIPTION OF THE SEVERAL VIEWS OF THE DRAWINGS

The following detailed description of the preferred embodiments of the present invention can be best understood when read in conjunction with the following drawings in which:
Figure 1A is a cross-sectional side view of a multi-layer dressing according to the present invention;
Figure 1B is a cross-sectional side view of the dressing of Figure 1A with a peel-off backing layer removed;
Figure 1C is a cross-sectional side view of another embodiment of the dressing including additional layers;
Figure 1D is a cross-sectional side view of yet a further embodiment of the dressing with a ring of adhesive for the adhesive layer;
Figure 2 is a graph illustrating % Protease B enzyme released from PSA/PVA formulations;
Figure 3 is a graph illustrating mg of Enzyme released from varying levels of LG12-containing PSA 7-4602 / PVA patches;
Figure 4 is a graph illustrating % Protease B enzyme released from 2220 formulations;
Figure 5 is a graph illustrating % Protease B enzyme released from 9090 formulations;
Figure 6 is a graph illustrating the effect of glycerin on the % Protease B enzyme released from PSA / PVA formulations;
Figure 7 is a graph illustrating the effect of processing on the release of the LG12 enzyme;
Figure 8 is a graph illustrating mg of LG12 released from PSA 7-4602 / PVA / colloidal silver patches;
Figure 9 is an illustration representing LG12 enzyme release from a PSA / PVA / colloidal silver formulation on a skim milk plate in 24 hours;
Figure 10 is an illustration representing LG12 enzyme release from a PSA / PVA / DC 5700 formulation on a skim milk plate in 24 hours;
Figure 11 is a graph illustrating mg of LG12 released from PSA 7-4602 + PVA patch formulation (10-day incubation at 42°C);
Figure 12 is a graph illustrating mg of LG12 released from PSA 7-4602 + PVA patch formulation (20-day incubation at 42°C); and
Figure 13 is a graph illustrating % Enzyme Released from a PVA + PSA 7-4602 patch formulated with varying levels of DC 3563.

### DETAILED DESCRIPTION OF THE INVENTION

In accordance with the present invention, a controlled-release composition may be used in a variety of topical dressings that may be applied to skin, wounded tissue, and diseased tissue. The topical dressings are multi-layered and allow the active agents to be released and applied to the underlying skin, wounded tissue, and diseased tissue. Additionally, the composition may be used to form ointments, and the ointments allow the active agents to be released and applied to the underlying skin, wounded, or diseased tissue.

A topical dressing shall be understood as referring to any of the various types of coverings that are suitable for application directly to the substrate, e.g. skin, wounded tissue, or diseased tissue for absorption of secretions, protection of the tissue from trauma, administration of an active agent to the tissue, protection of the tissue from the environment, to stop bleeding, to maintain or provide a moist environment, and combinations thereof. For example, the topical dressing may be in the form of films, patches, bandages, gels, ointments, and other semi-solid compositions that, when applied to the skin, dry to form films or coatings on the skin. The topical dressing is multi-layered in the sense that it includes a layer of the controlled-release composition and at least one additional layer, such as an adhesive layer. However, without a multi-layered dressing, the controlled-release composition by itself can form the film on the substrate on functional as a suitable topical application or 'dressing'.

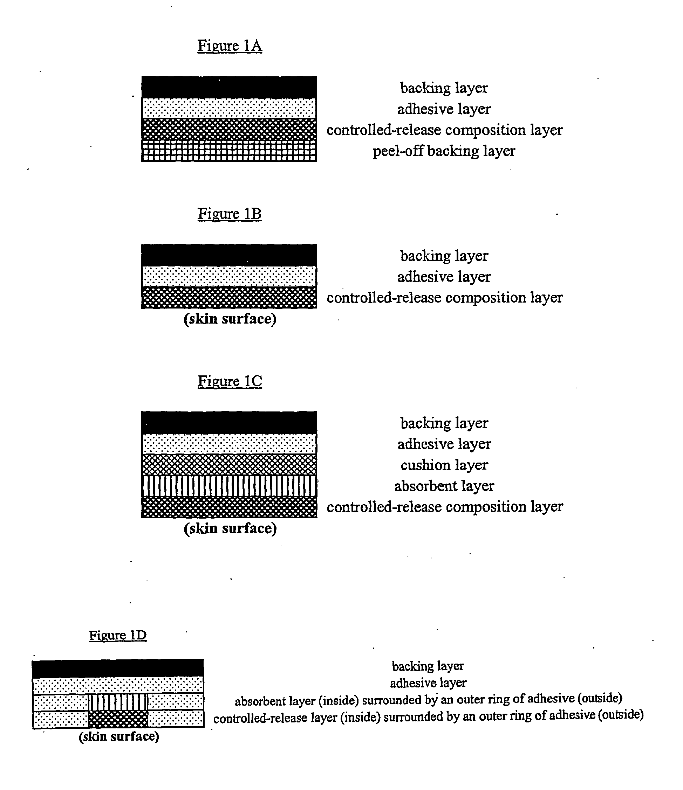

Various forms of the multi-layer dressing are disclosed in Figures 1A-1D. The dressing may include as many as about five layers. The various layers are labeled in Figures 1A-1D. The adhesive layer (light dots) enhances binding to provide better attachment of the controlled-release composition layer (dark cross-hatch). The controlled-release composition layer could be covered by an absorbent layer (vertical lines) to adsorb exudates and this layer could be overlaid by a cushion or cushioning layer (light cross-hatch). A backing layer (solid black) is occlusive to liquid water. Also, referring specifically to Figure 1A, before application or adherence to the substrate, i.e., the skin surface, the dressing may also include a peel-off backing layer which is removable. Further, as shown in Figure 1D, various layers, such as the adhesive layer, could be formed as a ring surrounding other layers.

Ointment shall be understood as referring to any suitable semi-solid preparation for external application, such as to skin, wounded tissue, and diseased tissue.

The present invention includes a controlled-release composition, essentially an emulsion that has been specifically processed and an active agent, for topical application to a substrate. The present invention also includes a method of delivering the active agent to the substrate. As eluded to above, the controlled-release composition, which is hereinafter simply referred to as the composition, includes the emulsion and the active agent. The active agent is incorporated into the emulsion.

Emulsion shall be understood as referring to a temporary or permanent dispersion of one liquid phase within a second liquid phase. Generally one of the liquids is water or an aqueous solution, and the other is oil or other water-immiscible liquid. Consequently, the continuous or external phase in a water-in-oil emulsion (W/O) is oil or other water-immiscible liquid. The continuous phase in an oil-in-water (O/W) emulsion is water or an aqueous solution. In accordance with the present invention, the controlled release compositions comprise an oil-in-water emulsion. For the descriptive purposes of the present invention, the term external phase is frequently used interchangeably with hydrophobic phase, and the term internal phase is frequently used interchangeably with hydrophilic phase. Advantageously, the active agent and, if present, the surfactant can be added to the emulsion during various emulsification steps that are undertaken to provide the emulsion or after the emulsion has been provided in a post-add situation without effecting the release profile of the active agent or the overall stability of the emulsion.

Controlled-release shall be understood to means that release kinetics are engineered into the system such that the active agent is released in a manner controlled by the system itself or its surroundings. The agent is not all released within a short period of time, e.g. less than about two hours, but rather is slowly released from a dressing in the presence of a trigger such as moisture over time, e.g about 4-6 hours, about 4-12 hours, about 4-18 hours, about 4-24 hours, about 4-36 hours, about 4-48 hours. A controlled-release is equivalent to a sustained-release.

Emulsion shall be understood as referring to a temporary or permanent dispersion of one liquid phase within a second liquid phase and encompasses W/O and O/W emulsions. Generally one of the liquids is water or an aqueous solution, and the other is an oil or other water-immiscible liquid. The first liquid is generally referred to as the continuous or external phase. Emulsions can be further classified as either simple emulsions, wherein the dispersed liquid or internal phase is a simple homogeneous liquid, or a more complex emulsion, wherein the dispersed liquid phase is a heterogeneous combination of liquid or solid phases, such as a double emulsion or a multiple-emulsion.

The emulsion is formed by mixing the internal and external phases in any suitable manner to form the preparations of the present invention such as high-shear processing. A preferred method is the mechanical inversion of a water-in-oil (W/O) emulsion as described additionally below, and the formed O/W emulsion may or may not contain lipophilic solvents. Mechanical inversion is also referred to in the art as mechanical inversion emulsification. The W/O emulsion, which is the basis for the O/W emulsion prior to mechanical inversion, includes a silicone component and a surfactant, preferably in a homogenous oil phase, and also includes water. The O/W emulsion is an embodiment that may be used in the dressings of the present invention. The dressing resulting from this emulsion is stable over time.

The silicone component of the emulsion, may be a hydrophobic or hydrophilic liquid, semi-solid (e.g. wax, gum), or solid. Regardless of whether the silicone component is itself hydrophobic or hydrophilic, the silicone component is contained within the hydrophobic phase of the emulsion. However, it is also possible that the silicone component be present within the hydrophilic phase of the emulsion. Furthermore, it is also possible that hydrophobic silicone components include some hydrophilic substituents and that hydrophilic silicone components include some hydrophobic substituents.

Preferably, the silicone component is a pressure sensitive adhesive (PSA) that is the reaction product of a hydroxy endblocked polydimethylsiloxane polymer and a hydroxy functional silicate resin. Preferably, the hydroxy functional silicate resin is a trimethylsiloxy and hydroxy endblocked silicate resin. The polymer and resin react in a condensation reaction to form the PSA. Although the PSA is most preferred, other forms of the silicone component include a silicone gum, a silicone rubber, a silicone elastomer, a silicone resin, high molecular weight silicones, or mixtures thereof these components. These other forms of the silicone component are possible because they form a film. Along with the active agent, the PSA functions as a bio-adhesive. The advantage of using the PSA as the silicone component is the substantivity that the PSA provides. This substantivity is particularly advantageous in human and veterinary applications that require significant substantivity for the active agent to provide sustained therapeutic effects.

The silicone components that are emulsified according to the mechanical inversion process, specifically the PSA, the silicone gum, the silicone rubber, the silicone elastomer, the silicone resin, and the high molecular weight silicones in the absence of a lipophilic solvent have viscosities up to 5,000,000,000 (5 billion) centipose (cP), preferably of at least 200,000,000 (200 million) centipose (cP) to 2,000,000,000 (2 billion) centipose (cP), and most preferably of at least 1,000,000,000 (1 billion) centipose (cP).

For purposes of this invention, the terms silicone rubber and silicone elastomer are synonymous, at least to the extent that both silicone components are capable of elongation and recovery. In contrast, silicone gums are capable of being stretched, but they do not generally snap back. Silicone gums are the high molecular weight, generally linear, polydiorganosiloxanes that can be converted from their highly viscous plastic state into a predominately elastic state by crosslinking. Silicone gums are often used as one of the main components in the preparation of silicone rubbers and silicone elastomers.

Silicone emulsions are aqueous emulsions of silicone elastomer particles. Removal of water from these emulsions results in either a silicone elastomeric film or particles of silicone elastomer. These emulsions can be prepared by emulsifying reactive silicone polymers and other ingredients such as catalysts or crosslinking compounds in water followed by a suitable vulcanizing (cure or crosslinking) step. Depending upon the emulsification conditions used, these elastomer emulsions can also be made with mean particle sizes that range from approximately 0.10um to 50um. Silicone elastomer emulsions can be considered to include compositions of the type described in United States patent N0. 6,497,894 (issued December 24, 2002) and United States Patent No. 5,321,075 (issued June 14, 1994) and United States Patent No. 4,248,751 (issued February 3, 1981).

For purposes of this invention therefore, silicone gum can be considered to include compositions of the type described in United States Patent No. 3,692,737 (issued September 19, 1972), United States Patent No. 4,152,416 (issued May 1, 1979), United States Patent No. 4,885,129 (issued August 8, 1989), and United States Patent No. 5,057,240 (issued October 15, 1991).

Silicone rubbers and silicone elastomers can be considered to include compositions of the type described in United States Patent No. 4,882,377 (issued November 21, 1989), United States Patent No. 5,654,362 (issued August 5, 1997), United States Patent No. 5,994,459 (issued November 30, 1999), and United States Patent No. 6,015,858 (issued January 18, 2004).

Silicone resins can be considered to include compositions of the type described in United States Patent No. 2,676,182 (issued April 20, 1954), United States Patent No. 4,310,678 (issued January 12, 1982), United States Patent No. 4,423,095 (issued December 27, 1983), and United States Patent No. 5,356,585 (issued October 18, 1994).

The silicone resins of the subject invention may also be considered to include *MQ* resins. The acronym *MQ* as it relates to silicone resins is derived from the symbols M, D, T, and Q each of which represent a functionality of different types of structural units which may be present in silicone resins containing siloxane units joined by ≡Si-O-Si≡ bonds. The monofunctional (M) unit represents (CH₃)₃SiO_{1/2} and the difunctional (D) unit represents (CH₃)₂SiO_{2/2}. The trifunctional (T) unit represents CH₃SiO_{3/2} and results in the formation of branched linear siloxanes. The tetrafunctional (Q) unit represents SiO_{4/2}, which results in the formation of crosslinked and resinous silicone compositions. Hence, MQ is used when the siloxane contains all monofunctional M and tetrafunctional Q units, or at least a high percentage of M and Q units such as to render the silicone resinous.

Silicone resins useful herein are non-linear siloxane resins having a glass transition temperature (Tg) above 0°C. Glass transition temperature is the temperature at which an amorphous material such as a higher silicone polymer changes from a brittle vitreous state to a plastic state. Thin silicone resin generally has the formula R'ₐSiO_{(4-a)/2} wherein R' is a monovalent hydrocarbon group with 1-6 carbon atoms or a functionally substituted hydrocarbon group with 1-6 carbon atoms, and a has an average value of 1-1.8. The silicone resin will preferably include monofunctional (M) units R''₃SiO_{1/2} and tetrafunctional (Q) units SiO_{4/2}, in which R" is the monovalent hydrocarbon group having 1-6 carbon atoms, most preferably the methyl group. Typically, the number ratio of M groups to Q groups will be in the range of 0.5:1 to 1.2:1, so as to provide an equivalent wherein a in the formula R'ₐSiO_{(4-a)/2} has an average value of 1.0-1.63. Preferably, the number ratio is 0.6:1 to 0.9:1. Most preferred are silicone MQ resins in which the number of Q units per molecule is higher than 1, preferably higher than 5.

The silicone resin may also contain 1-5 percent by weight of silicon-bonded hydroxyl radicals such as a dimethylhydroxysiloxy unit (HO)(CH₃)₂SiO_{1/2}. If desired, the silicone resin may contain minor amounts of difunctional (D) units and/or trifunctional (T) units. The silicone resin may include (i) silicone resins of the type MₓQ_{y} where x and y have values such that the silicone resin contains at least more than 5 Q units per molecule; (ii) silicone resins of the type MₓT_{y} where x and y have values such that the silicone resin contains at least more than 5 T units per molecule; and (iii) silicone resins of the type MₓD_{y}TₚQ_{q} where x, y, p, and q have values such that the sum of Q and T units is at least more than 5 units per molecule, and the number of D units varies from 0-100.

As set forth above, the emulsion may include a surfactant. Surfactant shall be understood as referring to a surface-active agent added to a suspending medium to promote uniform and maximum separation of immiscible liquids or liquids and extremely fine solid particles, often of colloidal size. As is understood by those skilled in the art, surfactants are amphiphilic molecules that have polar head groups and nonpolar chains. As such, the surfactants accumulate at the interfaces of the hydrophilic and hydrophobic phases and the polar heads orient toward the hydrophilic phase and the nonpolar chains orient toward the hydrophobic phase. Surfactants promote wetting, efficient distribution of immiscible liquids, droplets, or fine solid particles in a liquid dispersing medium and stabilization against particle aggregation. The surfactant is generally added in the dispersing medium in amount sufficient to provide complete surface coverage of the particle surface. The surfactant may be an anionic surfactant, cationic surfactant, nonionic surfactant, amphoteric surfactant, or a mixture of these surfactants.

Representative examples of suitable anionic surfactants include alkali metal salts of higher fatty acids, alkylaryl sulphonates such as sodium dodecyl benzene sulphonate, long chain fatty alcohol sulphates, olefin sulphates and olefin sulphonates, sulphated monoglycerides, sulphated esters, sulphonated ethoxylated alcohols, sulphosuccinates, alkane sulphonates, phosphate esters, alkyl isethionates, alkyl taurates, and alkyl sarcosinates. One example of a preferred anionic surfactant is sold commercially under the name Bio-Soft N-300. It is a triethanolamine salt of dodecylbenzene sulfonic acid marketed by the Stephan Company, Northfield, Illinois.

Representative examples of suitable cationic surfactants include alkylamine salts, quaternary ammonium salts, sulphonium salts, and phosphonium salts.

One example of a preferred cationic surfactant is cetyltrimethylammonium chloride sold commercially under the name Ammonxy CETAC 30 marketed by the Stephan Company. Another example is a quaternary ammonium-functional silane sold commercially under the name DC 5700 marketed by the Aegis Company.

Representative examples of suitable nonionic surfactants include condensates of ethylene oxide with long chain fatty alcohol or fatty acids such as a C₁₂₋₁₆ alcohol, condensates of ethylene oxide with an amine or an amide, condensation products of ethylene and propylene oxide, esters of glycerol, sucrose, sorbitol, fatty acid alkylol amides, sucrose esters, fluoro-surfactants, and fatty amine oxides. Representative examples of suitable amphoteric surfactants include imidazoline compounds, alkylaminoacid salts, and betaines.

Representative examples of suitable commercially available nonionic surfactants include polyvinyl alcohol (PVA or PVOH) (such as, for example, Mowiol^{®} 3-83 and 30-92 available from Clariant Corporation, Charlotte, N.C.) and polyoxyethylene fatty alcohols sold under the tradename BRIJ by Uniqema (ICI Surfactants), Wilmington, Delaware. Some examples are BRIJ 35 Liquid, an ethoxylated alcohol known as polyoxyethylene (23) lauryl ether, and BRIJ 30, another ethoxylated alcohol known as polyoxyethylene (4) lauryl ether. Some additional nonionic surfactants include ethoxylated alcohols sold under the trademark TERGITOL® by The Dow Chemical Company, Midland, Michigan. Some example are TERGITOL® TMN-6, an ethoxylated alcohol known as ethoxylated trimethylnonanol; and various of the ethoxylated alcohols, i.e., C₁₂-C₁₄ secondary alcohol ethoxylates, sold under the trademarks TERGITOL® 15-S-5, TERGITOL® 15-S-12, TERGITOL® 15-S-15, and TERGITOL® 15-S-40. Surfactants containing silicon atoms such as silicone polyethers can also be used.

Upon the providing of the emulsion, which includes the silicone component, and optionally the surfactant and the water, the active agent is incorporated, or dispersed, into the emulsion for delivery of the active agent to the substrate upon application of the emulsion to the substrate. Although the active agent may be in powder form or crystalline form, it is typically in liquid or viscous form. The active agent can be post-added into the emulsion whether or not it is combined with a hydrophilic carrier and/or hydrophilic component. Alternatively, the active agent can be incorporated during the steps to provide the emulsion.

Hydrophilic carrier shall be understood as referring to at least one component of a phase of the preparations of the present invention that acts as the solvent for the active agents. The hydrophilic carrier aids in the release of the active agent from the silicone matrices used in embodiments of the present invention.

Hydrophilic component shall be understood as referring to at least one component added to the mixture of the hydrophilic carrier and active agent in embodiments of the present invention. The hydrophilic component may aid in the release of the active agent from the silicone matrices used in embodiments of the present invention.

Active Agent shall be understood as referring to proteins, and in particular to enzymes.

Protein shall be understood as referring to natural, synthetic, and engineered enzymes such as oxidoreductases, transferases, isomerases, ligases, hydrolases; antibodies; polypeptides; peptides; hormones; cytokines; growth factors; and other biological modulators.

The active agents of the present invention are generally proteins, such as enzymes, that are incorporated into the hydrophilic carrier. The active agents may be hydrophilic. Enzymes suitable for incorporation in the dressing may be any enzyme or enzymes. Enzymes include, but are not limited to, commercially available types, improved types, recombinant types, wild types, variants not found in nature, and mixtures thereof. For example, suitable enzymes include hydrolases, cutinases, oxidases, transferases, reductases, hemicellulases, esterases, isomerases, pectinases, lactases, peroxidases, laccases, catalases, and mixtures thereof. Hydrolases include, but are not limited to, proteases (bacterial, fungal, acid, neutral or alkaline), amylases (alpha or beta), lipases, mannanases, cellulases, collagenases and mixtures thereof.

Lipase enzymes which may be considered to be suitable for inclusion in the preparations of the present invention include those produced by microorganisms of the Pseudomonas group, such as *Pseudomonas stutzeri* ATCC 19.154, as disclosed in British Patent 1,372,034; *Pseudomonas mendocina,* as described in U.S. Patent No. 5,389,536, and *Pseudomonas pseudoalcaligenes,* as disclosed in U.S. Patent No 5,153,135. Lipases further include those that show a positive immunological cross-reaction with the antibody of the lipase, produced by the microorganism *Pseudomonas fluorescens* IAM 1057. This lipase is available from Amano Pharmaceutical Co. Ltd., Nagoya, Japan, under the trade name Lipase P "Amano". Lipases include M1 Lipase® and Lipomax® (Gist-Brocades NV, Delft, Netherlands) and Lipolase® (Novozymes A/S, Bagsvaerd, Denmark). The lipases are normally incorporated in the silicone matrix at levels from about 0.0001% to about 2% of active enzyme by weight of the silicone matrix, or from about 0.001 mg/g to about 20 mg/g.

Proteases are carbonyl hydrolases which generally act to cleave peptide bonds of proteins or peptides. As used herein, "protease" means a naturally-occurring protease or a recombinant protease. Naturally-occurring proteases include .alpha.-aminoacylpeptide hydrolase, peptidylamino acid hydrolase, acylamino hydrolase, serine carboxypeptidase, metallocarboxypeptidase, thiol proteinase, carboxylproteinase and metalloproteinase. Serine, metallo, thiol and acid proteases are included, as well as endo and exo-proteases.

The protease can be of animal, plant, or microorganism origin. For example, the protease may be a serine proteolytic enzyme of bacterial origin. Purified or nonpurified forms of enzyme may be used. Protease enzymes produced by chemically or genetically modified mutants are included by definition, as are close structural enzyme variants. Particularly preferred by way of protease enzyme is bacterial serine proteolytic enzyme obtained from *Bacillus,* particularly subtilases, for example *Bacillus subtilis, Bacillus lentus, Bacillus amyloliquefaciens,* and/or *Bacillus licheniformis*. Suitable commercial proteolytic enzymes which may be considered for inclusion in the present invention compositions include Alcalase®, Esperase®, Durazym®, Everlase®, Kannase®, Relase®, Savinase®, Maxatase®, Maxacal®, and Maxapem® 15 (protein engineered Maxacal); Purafect®, Properase® (protein engineered Purafect) and subtilisin BPN and BPN'.

Protease enzymes also encompass protease variants having an amino acid sequence not found in nature, which is derived from a precursor protease by substituting a different amino acid sequence not found in nature, which is derived from a precursor protease by substituting a different amino acid for the amino acid residue at a position in said protease equivalent to positions equivalent to those selected from the group consisting of +76, +87, +99, +101, +103, +104, +107, +123, +27, +105, +109, +126, +128, +135, +156, +166, +195, +197, +204, +206, +210, +216, +217, +218, +222, +260, +265, and/or +274 according to the numbering of *Bacillus amyloliquefaciens* subtilisin, as described in U.S. Patent Nos. RE 34,606; 5,700,676; 5,972,682 and/or 6,482,628.

Exemplary protease variants include a subtilisin variant derived from *Bacillus lentus,* as described in U.S. Patent No. RE 34,606, hereinafter referred to as Protease A. Another suitable protease is a Y217L variant derived from *Bacillus amyloliquesfaciens,* as described in U.S. Patent No. 5,700,676, hereinafter referred to as Protease B. Also suitable are what are called herein Protease C, which is a modified bacterial serine proteolytic enzyme described in U.S. Patent No. 6,482,628; and Protease D, which is a modified bacterial serine proteolytic enzyme described in U.S. Patent No. 5,972,682. Also suitable is LG12 a *B. subtilis* as described in US Pat. No. 5,677,163.

Other proteases useful in the practice of this invention can be selected from the group consisting of Savinase®, Esperase®, Maxacal®, Purafect®, BPN', Protease A, Protease B, Protease C, Protease D, LG12 and mixtures thereof. Protease enzymes are generally present in the preparations of the present invention at levels from about 0.0001% to about 2% of active enzyme by weight of the silicone matrix, or from about 0.001 mg/g to about 20 mg/g.

It will be understood by those having skill in the art that the present invention is not limited to the enzymes listed above. It shall be further understood by those having skill in the art that one or more active agents including non-proteinaceous active agents such as anti-infection and biocide agents can be utilized in the topical preparations of the present invention.

The active agents, and any non-proteinaceous agents, may perform a variety of functions. For example, the matrix can release proteases and other enzymatic debriding agents topically for removal of necrotic tissues and general wound cleansing, clotting formation and clot removal enzymes, agents which generate peroxide, peracid, activated oxygen species, and anti-adhesion catalytic antagonists for self-sterilization, anti-infection, and acceleration of healing, and agents for skin treatment and the like.

Additionally, hydrophilic and/or amphiphilic excipients can be employed to stabilize or compatibilize the active agents, as well as assist in their release from the silicone matrix. Excipients can be liquid, semi-solid (e.g. wax, gum), or solid. Silicone excipients for use with the present invention can include silicone polyethers, silicone fluids, dimethicones, dimethicone copolyols, dimethiconols, silicone alkyl waxes, silicone polyamides and the like. Other possible excipients include, but are not limited to, silver, (poly)saccharide derivatives, acrylate derivatives, PVA derivatives, glycol, glycerol, glyceride derivatives, propylene glycol (PPG), polyethylene glycol, poloxamer, glycerin, alcohol, cellulosic derivatives, polyacrylic acids, alginate derivatives, chitosan derivatives, gelatin, pectin and polyhydric alcohol.

Also, various cosmetic, personal care, and cosmeceutical components may be included aside from the excipient or excipients. Examples of suitable cosmetic, personal care, and cosmeceutical components include, but are not limited to, alcohols, fatty alcohols and polyols, aldehydes, alkanolamines, alkoxylated alcohols (e.g. polyethylene glygol derivatives of alcohols and fatty alcohols), alkoxylated amides, alkoxylated amines, alkoxylated carboxylic acids, amides including salts (e.g. ceramides), amines, amino acids including salts and alkyl substituted derivatives, esters, alkyl substituted and acyl derivatives, polyacrylic acids, acrylamide copolymers, adipic acid copolymers, alcohols, aminosilicones, biological polymers and derivatives, butylene copolymers, carbohydrates (e.g. polysaccharides, chitosan and derivatives), carboxylic acids, carbomers, esters, ethers and polymeric ethers (e.g. PEG derivatives, PPG derivatives), glyceryl esters and derivatives, halogen compounds, heterocyclic compounds including salts, hydrophilic colloids and derivatives including salts and gums (e.g. cellulose derivatives, gelatin, xanthan gum, natural gums), imidazolines, inorganic materials (clay, TiO2, ZnO), ketones (e.g. camphor), isethionates, lanolin and derivatives, organic salts, phenols including salts (e.g. parabens), phosphorus compounds (e.g. phosphate derivatives), polyacrylates and acrylate copolymers, protein and enzymes derivatives (e.g. collagen), synthetic polymers including salts, siloxanes and silanes, sorbitan derivatives, sterols, sulfonic acids and derivatives and waxes.

The method of the subject invention includes more specific steps in order to provide the emulsion. In accordance with a preferred embodiment, a preparation is provided comprising an internal or non-miscible dispersed phase within an external or continuous phase. The hydrophobic phase comprises a silicone matrix, and the hydrophilic phase comprises a hydrophilic carrier containing at least one active agent. Additionally, the hydrophilic phase may further comprise any suitable hydrophilic component.

The hydrophilic phase may comprise any suitable hydrophilic carrier containing at least one active agent. In an embodiment according to the invention, the hydrophilic carrier is a liquid at relevant temperatures, and solid materials (for example sorbitol, manitol, lactose, sodium chloride and citric acid) dissolved in suitable solvent also may be used. For example, the active agent may be contained in a solution of propylene glycol (PPG), polyethylene glycol, poloxamer, glycerin, alcohol, polyhydric alcohol, water, or other suitable hydrophilic carrier.

The hydrophilic phase may further comprise a water soluble and hydrophilic component. The hydrophilic component generally does not serve as a solvent for the active agent. The hydrophilic component may enhance the release rate of the active agent from the silicone matrix and can include polyvinyl alcohol (PVA or PVOH) (such as, for example, Mowiol^{®} 3-83 and 30-92 available from Clariant Corporation, Charlotte, N.C.). The hydrophilic phase solution can include up to about 50 or more wt.% PVA solution in water. However, it is understood that both higher molecular weight and increased concentration of PVA result in a higher viscosity of the final composition. In an embodiment according to the invention, the hydrophilic component can also be a water-thickening agent diluted in water such as cellulosic derivatives (such as carboxymethylcellulose, methylcellulose, sodium carboxymethyl cellulose, hydroxypropylcellulose, hydroxypropylmethylcellulose), polyacrylic acids, alginate derivatives, chitosan derivatives, gelatin, pectin, polyethylene glycol, propylene glycol, glycerol and other suitable hydrophilic molecules and macromolecules in which the active agent may or may not be soluble. Such molecules include hydrophilic macromolecules.

The emulsion is formed by mixing the internal and external phases in any suitable manner to form the preparations of the present invention such as high-shear processing. Preferably, the W/O emulsion is mechanically-inverted into an O/W emulsion. This mechanical inversion is preferably accomplished by applying a high shear to the W/O emulsion. Suitable high-shear equipment include a high-intensity mixer, homogenizer, colloidal mill, Sonolator, Microfluidizer, ultrasonic processor, change can mixer, and a generic dental mixer such as Hauschild SpeedMixer ™ supplied by Flacktek. The preferred mixing device (i.e. dental mixer) consists of a mixer enclosed in a housing and having a motorized arm that rotates about a first axis of rotation, and a basket arranged to rotate about a second axis of rotation, in the opposite direction while the arm is rotating. During operation the basket rotates around an axis in one direction while it is simultaneously rotating (oppositely) in a planaterary motion around another axis. A container holding the substances to be mixed is placed in the basket and the mixer is energized for a period of time, which is controlled by an electronic timer. As the mixing is very efficient, the normal mixing periods are typically on the order of 20 seconds. This mixer is sold commercially by the name of SpeedMixer ^{™} by Flacktek, Inc., Landrum, SC. A description of this mixer may be found in US Patent No. 6,755,565 (issued June 29,2004).

Inversions generally occur when the continuous phase of a dispersion becomes the dispersed phase, or vice versa. Phase inversions in liquid/liquid dispersions are categorized as either catastrophic inversions or transitional inversions. Catastrophic inversions are caused by simply changing the phase ratio until there is a high enough ratio of the dispersed phase that it becomes the continuous phase. Transitional inversions occur when the affinity of the surfactant for the two phases is altered in order to cause the inversion.

After inversion, the O/W emulsion may then be diluted with additional water. If added, the additional water is typically added after a desired particle size for the silicone component has been reached. The droplet size of the internal phase may vary. For example, the droplet size in the preferred embodiment may be from about 0.01 µm up to about 1000 µm, while the most preferred embodiment is from about 0.1 µm up to about 0.5 µm.

The solids content may be selectively varied to achieve a target viscosity for ideal application of the emulsion to the substrate or to effect the rate of delivery of the active agent to the substrate.

As alluded to above, the active agent can be incorporated during the steps that are undertaken to provide the emulsion. More specifically, the active agent may be incorporated into the emulsion by incorporating the active agent along with the step of adding the water to the emulsion containing the continuous phase and the dispersed phase. The emulsion can contain other additives including, but not limited to, biocides (e.g. DC 5700), silver, thickeners, freeze-thaw stabilizers and electrically conductive additives, such as an ionic species, to make a conductive emulsion that can be used as electrodes in electrophoretic applications.

The method of delivering the active agent to the substrate further includes the step of applying the emulsion to the substrate to deliver the active agent to the substrate. Upon application of the emulsion, which contains the active agent, and upon exposure of the substrate to air, the solvent leaves the emulsion and a film is formed on the substrate. The film contains the active agent.

In sum, the method provides at least one layer of the dressing, then provides the emulsion, oil-in-water (or water-in-oil which is inverted to oil-in-water), then incorporates the active agent comprising the protein into the emulsion to establish the controlled-release composition, and the controlled-release composition is applied the layer to form a controlled-release layer of the dressing. Upon processing, the controlled-release composition may be dried such that the controlled-release layer is free of water. Free of water can be understood to include free of all water or free of all water, with the exception of any water inherently bound to the enzyme.

In embodiments where the substrate is skin, the emulsion is applied to the skin to deliver the active agent to the skin. The emulsion may be applied, i.e., rubbed or coated, directly onto the skin. Alternatively, the emulsion may be incorporated into a bandage or patch dressing prior to application to the substrate, i.e., to the skin.

The controlled-release composition according to this invention is capable of delivering performance properties such as adhesion, controlled tack, controlled lubrication, shear reduction, cushioning, water resistance, barrier properties, maintenance or provision of a moist wound environment, and scar-reduction. This controlled-release composition has substantivity to the skin and other substrates. In addition, an adhesive substance can be applied to a transdermal patch to improve adhesion if desired. The significant substantivity of the composition is particularly advantageous when delivery of the active agent is required over an extended period of time. Simply stated, the controlled-release composition is topically applied to the substrate where the film remains over the extended period of time. When the substrate is skin, the substantivity is important due to the presence of certain body oils and especially upon application to hairy skin. The composition also has substantivity to wet substrates such as wounds.

The topical dressing may be a liquid, semi-solid, or solid.

Since the topical dressing is understood as referring to any of the various types of coverings, a liquid or semi-solid dressing may be in the form of an ointment, gel, foam, and a low viscosity fluid. Such dressings could be packaged and delivered from a tube, syringe, stick, pump, spray, or a wipe and combinations thereof. The liquid form can remain as a liquid, such as an ointment dressing, or solidify during the formation of a film due to evaporation or cross-linking such as a liquid bandage.

A solid dressing has a three dimensional form such as an adhesive strip, bandage, putty, or a single or multi-layer film or membrane (e.g. transdermal patch). As illustrated in Figure 1, a three-dimensional solid dressing may include an adhesive, controlled-release composition, absorbent, cushion, or a backing material and combinations thereof. The solid dressing may utilize an outer adhesive layer that extends beyond the outer margin of the controlled-release composition layer, and/or adhesive material may be positioned on the skin facing controlled-release composition layer, but along the outer margins of this layer. The multi-layer solid dressing may be in the form of continuous or discrete layers, dots, adhesive rim, or pattern coated network layer including open space and combinations thereof. The solid dressing may have any type of shape, thickness, and size. It can be highly flexible or rigid. It can be self-adhering (e.g. a full adhesive surface or adhesive rim) or require a secondary dressing or bandage to remain in place. It can be self-supported, impregnated into a textile (e.g. gauze, Dacron net, knitted fabric), and/or reinforced with an additional backing material. A natural (e.g. collagen, alginate, cellulose) or synthetic (e.g. plastic and elastomeric films) backing material may be a nonwoven material or a knitted textile, transparent or opaque, perforated, plain, embossed, or cellular (e.g. foam) and combinations thereof. For example, plastic and elastomeric films include semi-occlusive polyurethanes, polyethylene, and silicone membrane, such as Dow Corning® 7-4107. The controlled-release composition is composed of preparations comprising silicone matrices and hydrophilic carriers that provide controlled-release of active agents. The continuous or discrete layers within a multi-layer solid dressing may provide multiple functions. For example, a controlled-release composition may also have adhesive, absorbent, cushion, or barrier properties and combinations thereof. The absorbent layer absorbs exudate fluids from wounds. Cushioning layers provide padding over wounds, such as diabetic foot ulcers, to prevent re-injury. The backing layer may be occlusive to liquids and provide structural support for the dressing. A solid dressing may be constructed using any type of process to transform and give shape to liquid or plastic materials including coating, casting, injection-molding, and extrusion. The final device may be made by punching it into a multi-layer sheet, molding it directly into the final packaging (e.g. blister), or assembling it from distinct pieces and combinations thereof.
In order that the invention may be more readily understood, reference is made to the following examples, which are intended to be illustrative of the invention, but are not intended to be limiting in scope.

### EXAMPLE 1

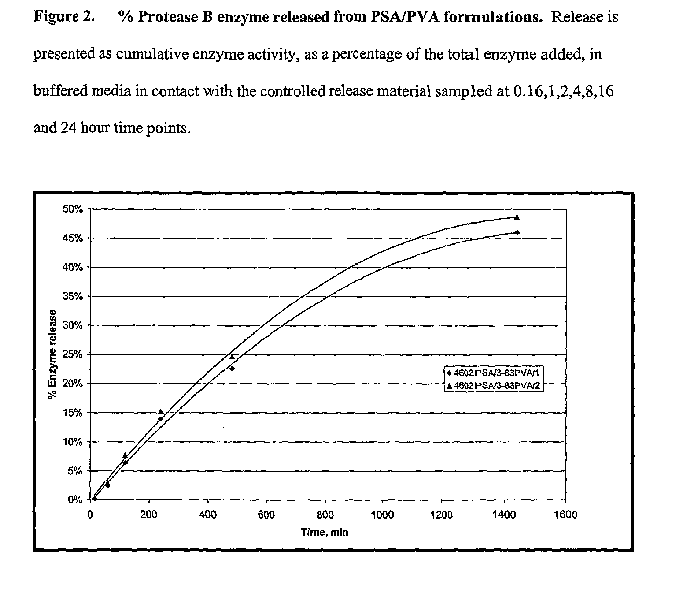

This experiment was conducted to evaluate the sustained release of Protease B enzyme from a silicone matrix. First the hydrophilic phase was prepared by mixing 8.71 g of hydrophilic carrier PVA solution (40% Mowiol 3-83 in water) with 0.767 ml of Protease B enzyme 42 mg/ml stock solution. Then, 20.43 g of the silicone phase was added to this mixture. The silicone matrix in this case was Dow Corning® PSA 7-4602 a pressure sensitive adhesive. After each addition step the sample was mixed two times in a Houschild AM-501 dental mixer. The prepared emulsions were spreaded on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Franz Cell Assembly and N-succinyl-L-Ala-L-Ala-L-Pro-L-Phe-p-nitroanilide (suc-AAPF-pNA) assay for proteolytic activity. The Franz Cell body was filled with dissolution buffer (10 mM MES, 10 mM CaCl2, and 0.005% Tween 80 at pH 5.4) and a patch sample was attached on top of the cell. Samples from the cell were collected after 15 minutes, 1 hour, 2 hours, 4 hours, 8 hours and 24 hours. From each collected samples, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 2 illustrates the results of the enzyme release from this matrix with Protease B enzyme. A controlled-release of about 50% Protease B was observed over 24 hours.

### EXAMPLE 2

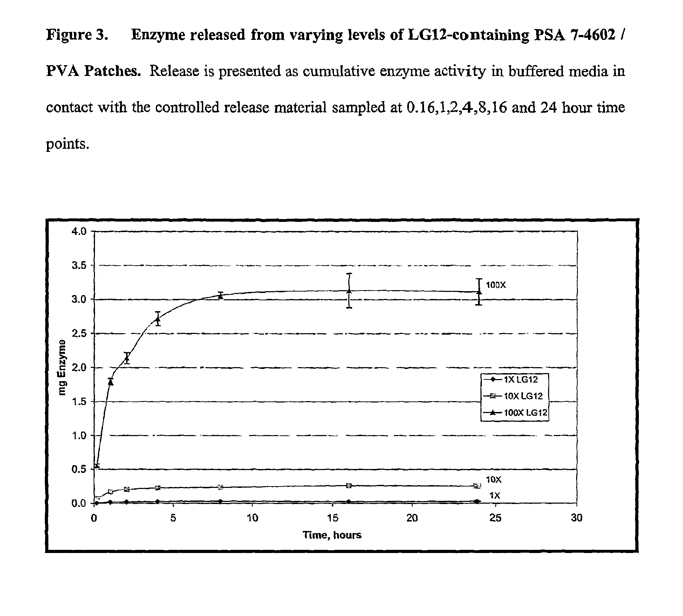

This experiment was conducted to evaluate the sustained release of LG12 protease from a silicone matrix using 1X, 10X and 100X enzyme loadings. First, the hydrophilic phase was prepared by mixing 13 g of hydrophilic carrier PVA solution (40% Mowiol 3-83 in water) with 3.192 ml LG-12 protease enzyme stock solution. Then, 30 g of silicone phase was added to this mixture. The enzyme stock solution was 0.4098 mg/ml in case of 1X, 4.098 mg/ml in case of 10X, and 40.98 mg/ml in case of 100X enzyme loading. The silicone matrix in this case was Dow Corning® PSA 7-4602 a pressure sensitive adhesive. After each addition step, the sample was mixed two times in a Houschild AM-501 dental mixer. The prepared emulsions were spreaded on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Hanson SR8 Plus Dissolution Tester and N-succinyl-L-Ala-L-Ala-L-Pro-L-Phe-p-nitroanilide (suc-AAPF-pNA) assay for proteolytic activity. The Hanson SR8 Plus Dissolution tester was filled with dissolution buffer (10 mM MES, 10 mM CaCl2, and 0.005% Tween 80 at pH 5.4) and a patch sample was placed inside of the vessel. Samples from the cell were collected after 10 minutes, 1 hour, 2 hours, 4 hours, 8 hours, 16 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 3 illustrates the results of the enzyme release from this matrix. Complete release of LG12 protease was observed over 24 hours. By increasing the enzyme load in the formulation, the enzyme release from the patches can be enhanced proportionally.

### EXAMPLE 3

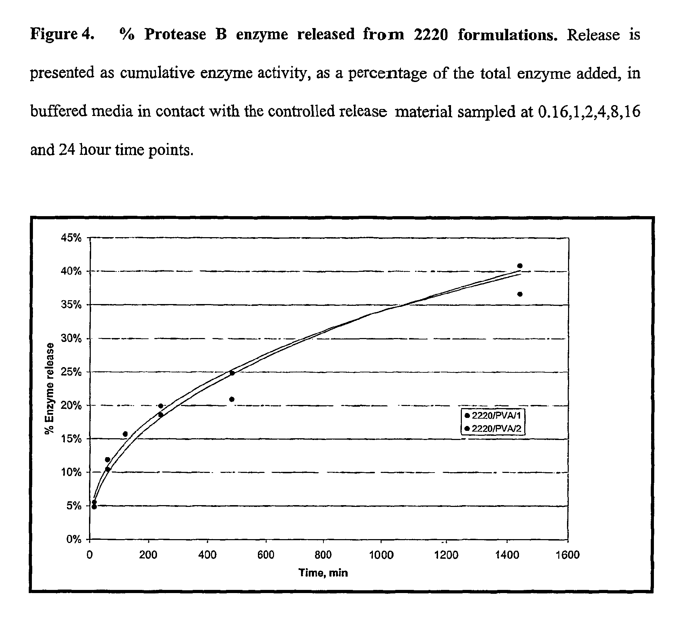

This experiment was conducted to evaluate the sustained release of Protease B enzyme from a silicone matrix. First, the hydrophilic phase was prepared by mixing 17.4 g of hydrophilic carrier PVA solution (10% Mowiol 30-92) with 0.42 g of Protease B enzyme 42 mg/ml stock solution. Then, 10.04 g of silicone phase was added to this mixture. The silicone matrix in this case was Dow Coming@ high molecular weight 2220 non-ionic emulsion. After each addition step, the sample was mixed two times in a Houschild AM-501 dental mixer. The prepared emulsions were spreaded on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Franz Cell Assembly and N-succinyl-L-Ala-L-Ala-L-Pro-L-Phe-p-nitroanilide (suc-AAPF-pNA) assay for proteolytic activity. The Franz Cell body was filled with dissolution buffer (10 mM MES, 10 mM CaCl2, and 0.005% Tween 80 at pH 5.4) and a patch sample was attached on top of the cell. Samples from the cell were collected after 15 minutes, 1 hour, 2 hours, 4 hours, 8 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 4 illustrates the results of the enzyme release from this matrix. A controlled-release of about 40% Protease B was observed over 24 hours.

### EXAMPLE 4

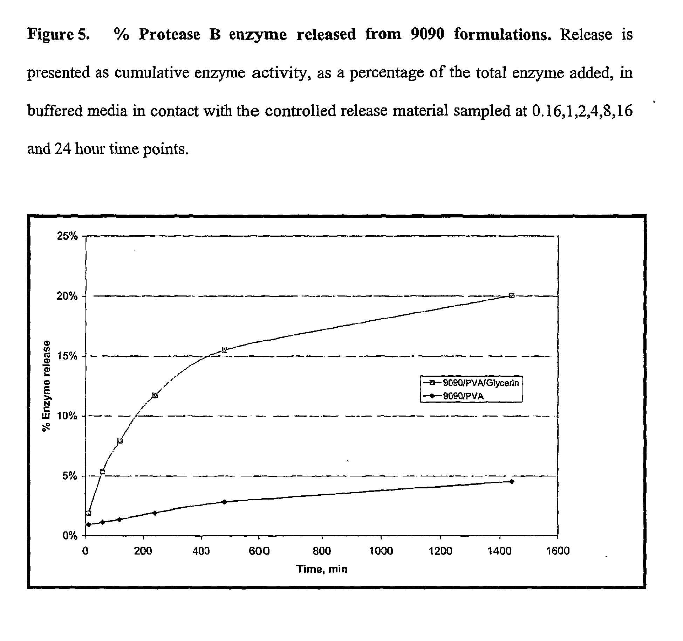

This experiment was conducted to evaluate the sustained release of Protease B enzyme from a crosslinked silicone matrix as well as assess the role of glycerin in the formulation. First, 0.49 g of Dow Corning® 1-3502 Si-H fluid was incorporated into 60 g of Dow Corning® SFD 128 vinyl silicone polymer. Subsequently, 10.08 g of 10% Mowiol (30-92) surfactant was added to the mixture until a high solid emulsion was formed. Then, 0.44 g of Pt catalyst was mixed into the formulation in emulsion form (Dow Corning® 2-1271). This formulation is analogous to a Dow Corning® 9090 Silicone Elastomer Emulsion. Then, the emulsion was diluted to 65% silicone solid content and 0.88 g of Protease B enzyme 42 mg/ml stock solution was added to this formulation. After each addition step, the sample was mixed two times in a Houschild AM-501 dental mixer. In the second formulation, 2% of the dry weight was replaced with glycerin and added in the same step as the PVA during the formulation. The prepared emulsions were spread on DC 7-4107 silicone membrane/ Polycarbonate substrate using a draw down bar made by Paul N. Gardner Company, Inc. The drawn film was allowed to dry and cured to a thin film on the substrate over 24 hours in ventilated hood. From the dried film, patches were cut out and analyzed for enzyme release activity. The samples were tested using Franz Cell Assembly and N-succinyl-L-Ala-L-Ala-L-Pro-L-Phe-p-nitroanilide (suc-AAPF-pNA) assay for proteolytic activity. The Franz Cell body was filled with dissolution buffer (10 mM MES, 10 mM CaCl2, and 0.005% Tween 80 at pH 5.4) and a patch sample was attached on top of the cell. Samples from the cell were collected after 15 minutes, 1 hour, 2 hours, 4 hours, 8 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 5 illustrates that glycerin enhances the rate of release of the Protease B enzyme from these matrices.

### EXAMPLE 5

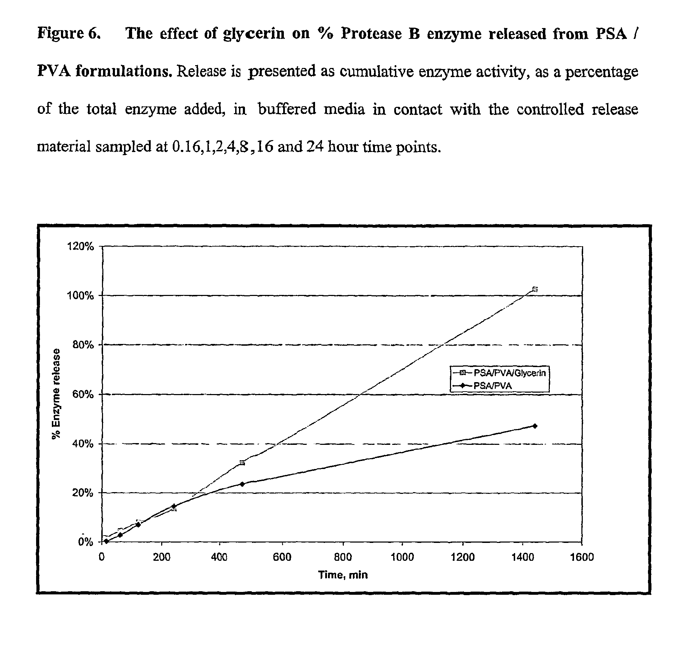

This experiment was conducted to evaluate the effect of glycerin on the sustained release of Protease B enzyme from a Dow Corning@ PSA 7-4602 a pressure sensitive adhesive silicone matrix. First, the hydrophilic phase was prepared by mixing 8.71 g of hydrophilic carrier PVA solution (40% Mowiol 3-83) with 0.767 ml of Protease B enzyme 42 mg/ml stock solution. Then, 20.43 g of silicone phase was added to this mixture. After each addition step, the sample was mixed two times in a Houschild AM-501 dental mixer. In the second formulation, 2% of the dry weight was replaced with glycerin and added in the same step as the PVA during the formulation. The prepared emulsions were spread on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Franz Cell Assembly and N-succinyl-L-Ala-L-Ala-L-Pro-L-Phe-p-nitroanilide (suc-AAPF-pNA) assay for proteolytic activity. The Franz Cell body was filled with dissolution buffer (10 mM MES, 10 mM CaCl2, and 0.005% Tween 80 at pH 5.4) and a patch sample was attached on top of the cell. Samples from the cell were collected after 15 minutes, 1 hour, 2 hours, 4 hours, 8 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 6 illustrates that glycerin enhances the rate of release of Protease B enzyme from these matrices.

### EXAMPLE 6

This experiment was conducted to evaluate the processing effect on the sustained release of LG-12 protease from a Dow Corning® PSA 7-4602 a pressure sensitive adhesive silicone matrix. First, the hydrophilic phase was prepared by mixing 13 g of hydrophilic carrier PVA solution (40% Mowiol 3-83) with 0.896 LG-12 protease enzyme stock solution. Then, 30 g of silicone phase was added to this mixture. In the first case, the silicone component was added in one step and, in the second case, the silicone was added in two steps. After each addition step, the sample was mixed two times in a Houschild AM-501 dental mixer. The prepared emulsions were spread on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Hanson SR8 Plus Dissolution Tester and N-succinyl-L-Ala-L-Ala-L-Pro-L-Phe-p-nitroanilide (suc-AAPF-pNA) assay for proteolytic activity. The Hanson SR8 Plus Dissolution tester was filled with dissolution buffer (10 mM MES, 10 mM CaCl2, and 0.005% Tween 80 at pH 5.4) and a patch sample was placed inside of the vessel. Samples from the cell were collected after 10 minutes, 1 hour, 2 hours, 4 hours, 8 hours, 16 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml.

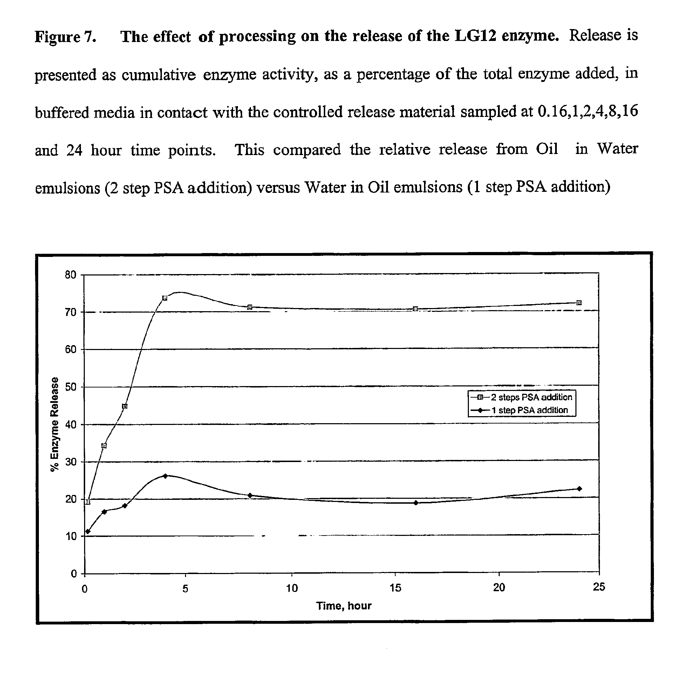

After examining the two types of samples in emulsion and film form, demonstrated differences were observed depending on whether the PSA silicone component was added in one or two steps to the hydrophilic phase. When the 7-4602 PSA was added in one step, the emulsion was thin and easy to spread on the 7-4107 membrane, while the dried film demonstrated good adhesion properties on the membrane. Less enzyme was released from this formulation as compared to the emulsion prepared using two steps. In contrast, when the 7-4602 was added in two steps, the resulting emulsion was thick and harder to spread on the 7-4107 membrane, while the dried film only adhered to the membrane with the aid of a 7-4600 PSA layer between the membrane and the film. The enzyme release from this formulation was greater than the enzyme release obtained from the film made using the one step process. Figure 7 illustrates the results of the enzyme release from these matrices.

Since the enzymatic release appeared to be dependent upon the addition steps in the formulation, a small amount of sample from each emulsion was mixed into water to test the nature of the emulsions. This investigation showed that when the 7-4602 PSA was added in one step, the emulsion did not combine with water. Comparatively, when 7-4602 PSA was added in two addition steps, the resulting emulsion dissipated into water. These results demonstrated that the first emulsion type was a W/O emulsion and the other sample was an O/W emulsion, respectively. Since an increased % LG12 protease was observed to release from an O/W emulsion, the processing was determined to have an effect on the resultant type of emulsion.

### EXAMPLE 7

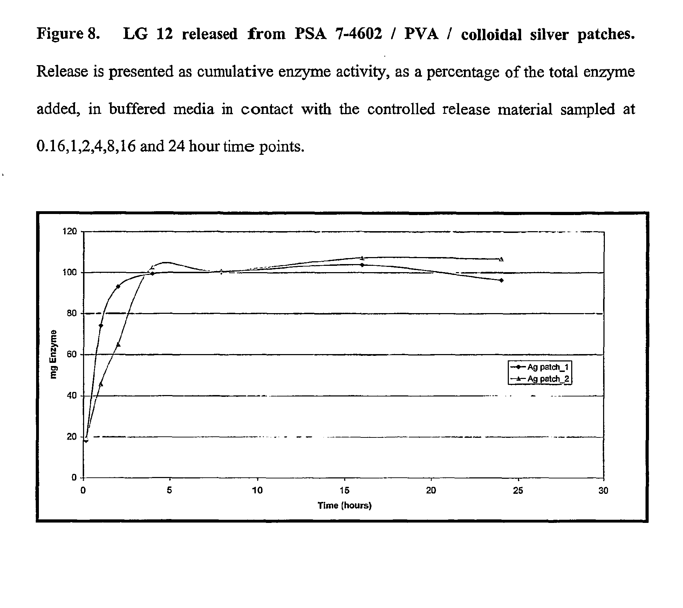

This experiment was conducted to evaluate the effect of colloidal silver on the sustained release of LG-12 protease from a Dow Coming@ PSA 7-4602 a pressure sensitive adhesive silicone matrix. The 40.98 mg/ml enzyme stock solution was diluted 10 fold in the colloidal silver solution (Colloidal Silver from Natural Immunogenics Corp. Miami, Florida). First, the hydrophilic phase was prepared by mixing 6.5 g of hydrophilic carrier PVA solution (40% Mowiol 3-83) with 1.596 ml LG-12 protease enzyme / colloidal silver solution. Then, 15 g of silicone phase was added to this mixture. After each addition step, the sample was mixed two times in a Houschild AM-501 dental mixer. The prepared emulsions were spread on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The samples were tested using Hanson SR8 Plus Dissolution Tester and N-succinyl-L-Ala-L-Ala-L-Pro-L-Phe-p-nitroanilide (suc-AAPF-pNA) assay for proteolytic activity. The Hanson SR8 Plus Dissolution tester was filled with dissolution buffer (10 mM MES, 10 mM CaCl2, and 0.005% Tween 80 at pH 5.4) and a patch sample was placed inside of the vessel. Samples from the cell were collected after 10 minutes, 1 hour, 2 hours, 4 hours, 8 hours, 16 hours and 24 hours. From each collected sample, aliquots were pipetted directly into a cuvette containing assay buffer (100 mM Tris and 0.005% Tween 80 at pH 8.6) and suc-AAPF-pNA substrate. Then, the enzyme activity was measured on a UV/Visible spectrometer, which gave the concentration of enzyme in the dissolution buffer in mg/ml. Figure 8 illustrates that the release of the LG12 protease from this matrix was complete after 4 hours.

In addition, the enzyme release was also followed on a skim milk plate.

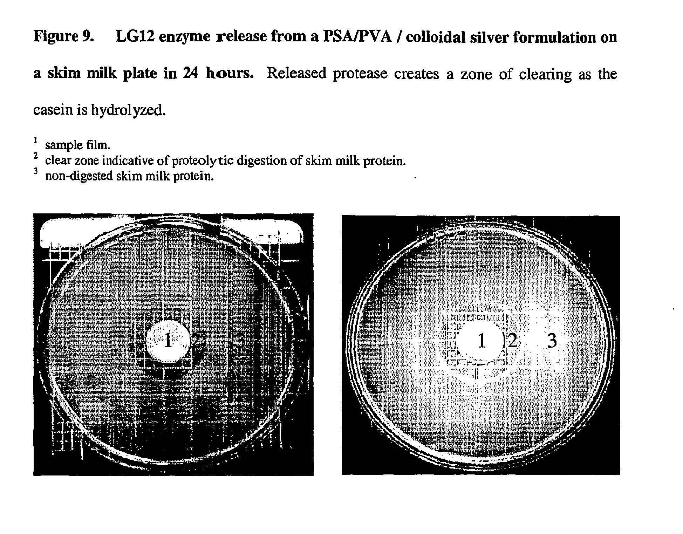

Skim milk plate preparation: In one bottle, skim milk powder was dissolved in water, and, in an other bottle, yeast extract, sodium chloride, and agar were mixed together. The bottles were autoclaved at 121°C for 15 minutes using Hirayama Autoclave. Then, they were allowed to cool to 45°C in a water bath. Afterward, the skim milk solution was added to the agar and the combined solution was poured into petri dishes and allowed to solidify. A small piece of the silicone matrix film containing the enzyme was cut out and pressed into the skim milk agar. The petri dish plate was allowed to incubate at 30±2°C. Figure 9 shows the enzyme released from the prepared patch on a skim milk plate after 24 hours incubation.

### EXAMPLE 8

This experiment was conducted to evaluate the effect of DC 5700 silane on the sustained release of LG-12 protease from a Dow Corning® PSA 7-4602 a pressure sensitive adhesive silicone matrix. First, the hydrophilic phase was prepared by mixing 6.54 g of hydrophilic carrier PVA solution (40% Mowiol 3-83) with 0.767 ml of Protease B enzyme 42 mg/ml stock solution. Then, 2.17 g of DC 5700 silane followed by 20.43 g of silicone phase was added to this mixture. After each addition step, the sample was mixed two times in a Houschild AM-501 dental mixer. The prepared emulsions were spread on DC 7-4107 silicone membrane/ Polycarbonate substrate using a draw down bar made by Paul N. Gardner Company, Inc. The drawn film was allowed to dry to a thin film on the substrate over 24 hours in a ventilated hood. From the dried film, patches were cut out and analyzed for enzyme release activity. The enzyme release was followed on a skim milk plate.

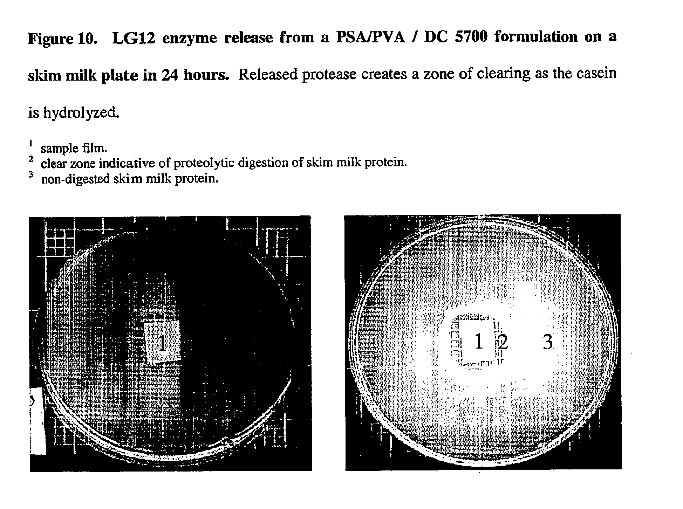

Skim milk plate preparation: In one bottle, skim milk powder was dissolved in water, and, in an other bottle, yeast extract sodium chloride, and agar were mixed together. The bottles were autoclaved at 121°C for 15 minutes using Hirayama Autoclave. Then they were allowed to cool down to 45°C in a water bath. Afterward, the skim milk solution was added to the agar and the combined solution was poured into petri dishes and allowed to solidify. A small piece of the silicone matrix film containing the enzyme was cut out and pressed into the skim milk agar. The petri dish plate was allowed to incubate at 30±2°C. Figure 10 shows the enzyme release from the prepared patch on a skim milk plate after 24 hours incubation. A small amount of enzyme was observed to release from this formulation.

### EXAMPLE 9

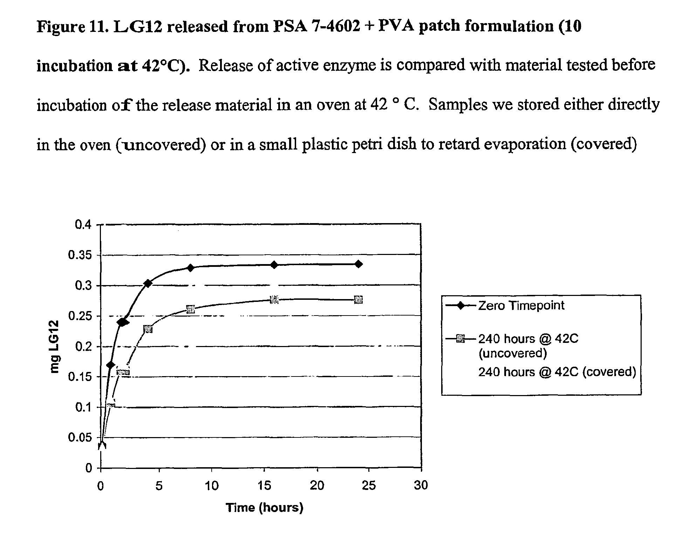

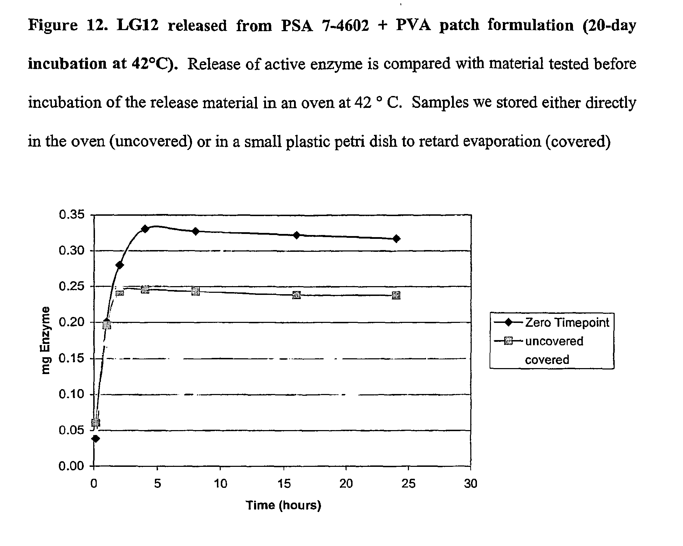

This experiment was conducted to evaluate enzyme stability in the silicone matrix. Dried PSA 7-4602 silicone + PVA films containing approximately 0.400 mg LG12 per gram dry weight were cut into 3.5 cm discs and incubated in a SYBRON Thermolyne Type 3700 culture incubator set at 42°C for 10 days and 20 days. Half of the number of the discs were placed inside an autoclave pouch to minimize moisture loss whereas the other half were left uncovered. Unincubated discs were weighed and loaded into a dissolution vessel of a Hanson SR8 Plus Dissolution Tester and used as control for this experiment. The dissolution buffer used was a 10mM MES pH 5.2 + 0.005% Tween-80 + 10mM calcium chloride. Fractions were collected after 10 minutes, 1,2,4,8,16 and 24 hours. These fractions were assayed for protease activity using N-succinyl-L-Ala-L-Ala-L-Pro-L-Phe-p-nitroanilide as the substrate to quantitate the amount of enzyme released from the silicone discs. As for the 42°C-incubated discs, the amount of remaining active enzyme released from the discs were measured using the same assay. Figures 11 and 12 illustrate the effect of moisture loss on enzyme stability in the silicone matrix.

### EXAMPLE 10

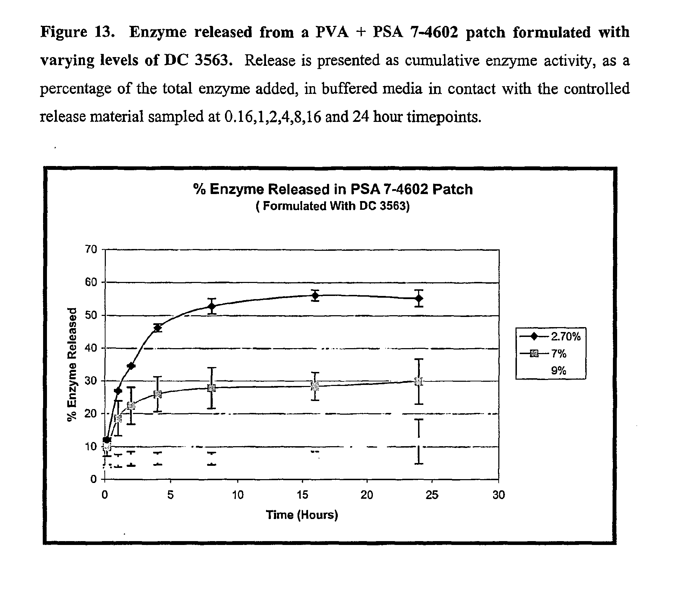

This experiment was conducted to create a more tacky silicone + PVA + enzyme film with the addition of DC 3563. Varying levels of DC 3563 were mixed with Dow Corning® PSA 7-4602, the silicone phase. This silicone mixture was then added to the hydrophilic phase which contains both the PVA solution (40% Mowial 3-83 in water) and the Protease B enzyme to make an emulsion. The emulsions were spread into films on a DC 7-4107 attached to a Mylar substrate using a metal spreader made by Paul N. Gardner Company, Inc. Dried films were cut into 3.5 cm diameter discs, weighed and loaded into a dissolution vessel of a Hanson SR8 Plus Dissolution Tester. The dissolution buffer used was a 10mM MES pH 5.2 + 0.005% Tween-80 + 10mM calcium chloride. Fractions were collected after 10 minutes, 1,2,4,8,16 and 24 hours. These fractions were assayed for protease activity using N-succinyl-L-Ala-L-Ala-L-Pro-L-Phe-p-nitroanilide as the substrate to quantitate the amount of enzyme released from the silicone discs. Figure 13 illustrates the effect of varying levels of DC 3563 on enzyme release.

## Claims

1. A controlled-release composition for topical application to a substrate, said composition comprising: an oil-in-water emulsion and an active agent comprising a protein incorporated into said emulsion, said emulsion having a hydrophilic phase comprising said active agent, water, and a carrier, and a hydrophobic phase comprising a silicone component.

2. A controlled-release composition as set forth in Claim 1, further comprising a surfactant between said hydrophilic and hydrophobic phases.

3. A controlled-release composition as set forth in Claim 1, wherein said carrier is selected from the group of glycerin, propylene glycol, polyethylene glycol, poloxamer, alcohol, polyhydric alcohol, water, polyvinyl alcohol, polyvinylpyrrolidone, and combinations thereof.

4. A controlled-release composition as set forth in Claim 1, wherein said carrier is in solution with said water.

5. A controlled-release composition as set forth in Claim 1, wherein said protein is an enzyme.

6. A controlled-release composition as set forth in Claim 5, wherein said enzyme is selected from the group of natural enzymes, synthetic enzymes, engineered enzymes, and combinations thereof.

7. A controlled-release composition as set forth in Claim 5, wherein said enzyme is selected from the group of oxidoreductases, transferases, isomerases, ligases, hydrolases, cutinases, oxidases, reductases, hemicellulases, esterases, pectinases, lactases, peroxidases, laccases, catalases, antibodies, polypeptides, peptides, hormones, cytokines, growth factors, biological modulators, and combinations thereof.

8. A controlled-release composition as set forth in Claim 5, wherein said enzyme comprises Protease A, Protease B, or LG12.

9. A controlled-release composition as set forth in Claim 2, further comprising a dispersing agent for dispersing said active agent.

10. A controlled-release composition as set forth in Claim 9, wherein said dispersing agent comprises a silicone-based surfactant different from said surfactant.

11. A controlled-release composition as set forth in Claim 1, wherein said silicone component is selected from the group consisting of a silicone gum, a silicone rubber, a silicone elastomer, a silicone resin, high molecular weight silicones, silicone emulsions, and combinations thereof.

12. A controlled-release composition as set forth in Claim 1, wherein said silicone component comprises a pressure sensitive adhesive.

13. A controlled-release composition as set forth in Claim 12, wherein said pressure sensitive adhesive comprises the reaction product of; a hydroxy endblocked polydimethylsiloxane polymer, and a hydroxy functional silicate resin.

14. A multi-layer dressing for topical application to a substrate, said dressing comprising: (A) a controlled-release layer formed from said controlled-release composition of Claim 1; (B) an adhesive layer disposed adjacent said controlled-release layer for adhering said dressing to the substrate; and (C) an additional layer selected from the group of a backing layer, a cushioning layer, an absorbent layer, a second adhesive layer, and combinations thereof.

15. A multi-layer dressing as set forth in Claim 14, wherein said controlled-release layer is adjacent the substrate and said additional layer is disposed adjacent said adhesive layer spaced from said controlled-release layer.

16. A multi-layer dressing as set forth in Claim 14, wherein said controlled-release layer is dry in said dressing such that said controlled-release layer is free of water after said controlled-release layer is formed by said controlled-release composition.

17. The use of the controlled release composition of any of Claims 1 to 13, for the manufacture of a multi-layer dressing for topical application to a substrate.

18. The controlled release composition of any of Claims 1 to 13, for use in medicine.

## Patentansprüche

1. Zusammensetzung zur kontrollierten Freisetzung, die dazu bestimmt ist, topisch auf ein Substrat aufgebracht zu werden, wobei die Zusammensetzung Folgendes enthält: eine Öl-in-Wasser-Emulsion und einen Wirkstoff, der ein Protein umfasst und der Emulsion beigemischt ist, wobei die Emulsion eine hydrophile Phase aufweist, die den Wirkstoff, Wasser und einen Trägerstoff umfasst, sowie eine hydrophobe Phase, die eine Silikonverbindung umfasst.

2. Zusammensetzung zur kontrollierten Freisetzung nach Anspruch 1, die weiterhin zwischen der hydrophilen und der hydrophoben Phase ein Tensid umfasst.

3. Zusammensetzung zur kontrollierten Freisetzung nach Anspruch 1, wobei der Trägerstoff aus der Gruppe von Glycerin, Propylenglykol, Polyethylenglykol, Poloxamer, Alkohol, Polyol, Wasser, Polyvinylalkohol, Polyvinylpyrrolidon und Kombinationen davon gewählt ist.

4. Zusammensetzung zur kontrollierten Freisetzung nach Anspruch 1, wobei der Trägerstoff in dem Wasser gelöst ist.

5. Zusammensetzung zur kontrollierten Freisetzung nach Anspruch 1, wobei das Protein ein Enzym ist.

6. Zusammensetzung zur kontrollierten Freisetzung nach Anspruch 5, wobei das Enzym aus der Gruppe der natürlichen Enzyme, der synthetischen Enzyme, der technisch veränderten Enzyme und Kombinationen davon gewählt ist.

7. Zusammensetzung zur kontrollierten Freisetzung nach Anspruch 5, wobei das Enzym aus der Gruppe der Oxidoreductasen, der Transferasen, der Isomerasen, der Ligasen, der Hydrolasen, der Cutinasen, der Oxidasen, der Reductasen, der Hemicellulasen, der Esterasen, der Pektinasen, der Lactasen, der Peroxidasen, der Laccasen, der Catalasen, der Antikörper, der Polypeptide, der Peptide, der Hormone, der Zytokine, der Wachstumsfaktoren, der biologischen Modulatoren und Kombinationen davon gewählt ist.

8. Zusammensetzung zur kontrollierten Freisetzung nach Anspruch 5, wobei das Enzym Protease A, Protease B oder LG12 umfasst.

9. Zusammensetzung zur kontrollierten Freisetzung nach Anspruch 2, die weiterhin ein Dispersionsmittel zur Dispersion des Wirkstoffs umfasst.

10. Zusammensetzung zur kontrollierten Freisetzung nach Anspruch 9, wobei das Dispersionsmittel ein Tensid auf Silikonbasis umfasst, das sich vom genannten Tensid unterscheidet.

11. Zusammensetzung zur kontrollierten Freisetzung nach Anspruch 1, wobei die Silikonverbindung aus der Gruppe gewählt ist, die aus einem Silikongummi, einem Silikonkautschuk, einem Silikonelastomer, einem Silikonharz, hochmolekularen Silikonen, Silikonemulsionen und Kombinationen davon besteht.

12. Zusammensetzung zur kontrollierten Freisetzung nach Anspruch 1, wobei die Silikonverbindung einen Haftklebstoff umfasst.

13. Zusammensetzung zur kontrollierten Freisetzung nach Anspruch 12, wobei der Haftklebstoff das Reaktionsprodukt von einem Polydimethylsiloxanpolymer mit Hydroxy-Endblock und einem hydroxyfunktionnellen Silikatharz umfasst.

14. Mehrschichtige Anordnung, die dazu bestimmt ist, topisch auf ein Substrat aufgebracht zu werden, wobei die Anordnung Folgendes umfasst: (A) eine Schicht zur kontrollierten Freisetzung, die aus der Zusammensetzung zur kontrollierten Freisetzung nach Anspruch 1 gebildet ist; (B) eine Klebstoffschicht, die derart angeordnet ist, dass sie an die Schicht zur kontrollierten Freisetzung angrenzt und dafür sorgt, dass die Anordnung am Substrat haftet; und (C) eine zusätzliche Schicht, die aus der Gruppe einer Trägerschicht, einer Dämpfungsschicht, einer absorbierenden Schicht, einer zweiten Klebstoffschicht und Kombinationen davon gewählt ist.

15. Mehrschichtige Anordnung nach Anspruch 14, wobei die Schicht zur kontrollierten Freisetzung an das Substrat angrenzt und die zusätzliche Schicht derart angeordnet ist, dass sie an die Klebstoffschicht angrenzt, räumlich von der Schicht zur kontrollierten Freisetzung getrennt.

16. Mehrschichtige Anordnung nach Anspruch 14, wobei die Schicht zur kontrollierten Freisetzung in der Anordnung in trockenem Zustand vorliegt und zwar derart, dass die Schicht zur kontrollierten Freisetzung wasserfrei ist, nachdem die Schicht zur kontrollierten Freisetzung aus der Zusammensetzung zur kontrollierten Freisetzung gebildet wurde.

17. Verwendung der Zusammensetzung zur kontrollierten Freisetzung nach einem beliebigen der Ansprüche 1 bis 13 zur Herstellung einer mehrschichtigen Anordnung, die dazu bestimmt ist, topisch auf ein Substrat aufgebracht zu werden.

18. Zusammensetzung zur kontrollierten Freisetzung nach einem beliebigen der Ansprüche 1 bis 13, die dazu bestimmt ist, in der Medizin verwendet zu werden.

## Revendications

1. Composition à libération contrôlée, destinée à l'application topique sur un substrat, ladite composition comprenant: une émulsion huile dans eau et un principe actif comprenant une protéine qui est incorporé à ladite émulsion, ladite émulsion ayant une phase hydrophile comprenant le principe actif, de l'eau et un support ainsi qu'une phase hydrophobe comprenant un composant de silicone.

2. Composition à libération contrôlée selon la revendication 1, comprenant en outre un agent tensioactif entre les phases hydrophile et hydrophobe.

3. Composition à libération contrôlée selon la revendication 1, ledit support étant choisi dans le groupe formé par le glycérol, le propylène glycol, le polyéthylène glycol, le poloxamère, l'alcool, le polyol, l'eau, l'alcool polyvinylique, la polyvinylpyrrolidone et leurs combinaisons.

4. Composition à libération contrôlée selon la revendication 1, ledit support étant en solution dans ladite eau.

5. Composition à libération contrôlée selon la revendication 1, ladite protéine étant une enzyme.

6. Composition à libération contrôlée selon la revendication 5, ladite enzyme étant choisie dans le groupe formé par les enzymes naturelles, les enzymes synthétiques, les enzymes modifiées par l'homme et leurs combinaisons.

7. Composition à libération contrôlée selon la revendication 5, ladite enzyme étant choisie dans le groupe formé par les oxydoréductases, les transférases, les isomérases, les ligases, les hydrolases, les cutinases, les oxydases, les réductases, les hémicellulases, les estérases, les pectinases, les lactases, les peroxydases, les laccases, les catalases, les anticorps, les polypeptides, les peptides, les hormones, les cytokines, les facteurs de croissances, les modulateurs biologiques et leurs combinaisons.

8. Composition à libération contrôlée selon la revendication 5, ladite enzyme comprenant de la protéase A, de la protéase B ou du LG12.

9. Composition à libération contrôlée selon la revendication 2, comprenant en outre un agent dispersant destiné à disperser le principe actif.

10. Composition à libération contrôlée selon la revendication 9, ledit agent dispersant comprenant un agent tensioactif à base de silicone qui est différent dudit agent tensioactif.

11. Composition à libération contrôlée selon la revendication 1, ledit composant de silicone étant choisi dans le groupe constitué par une gomme de silicone, un caoutchouc de silicone, un élastomère de silicone, une résine de silicone, des silicones à masse moléculaire élevée, des émulsions de silicone et leurs combinaisons.

12. Composition à libération contrôlée selon la revendication 1, le composant de silicone comprenant un adhésif sensible à la pression.

13. Composition à libération contrôlée selon la revendication 12, ledit adhésif sensible à la pression comprenant le produit de la réaction entre un polymère de type polydiméthylsiloxane pourvu d'un bloc terminal aux fonctions hydroxyle et une résine de silicate pourvue de groupes fonctionnels hydroxyle.

14. Dispositif multicouche destiné à l'application topique sur un substrat, ledit dispositif comprenant: (A) une couche à libération contrôlée formée à partir de ladite composition à libération contrôlée selon la revendication 1; (B) une couche d'adhésif disposée de façon à ce qu'elle jouxte ladite couche à libération contrôlée et destinée à assurer l'adhérence du dudit dispositif au substrat; et (C) une couche supplémentaire choisie dans le groupe formé par une couche de support, une couche d'amortissement, une couche absorbante, une deuxième couche d'adhésif et leurs combinaisons.

15. Dispositif multicouche selon la revendication 14, ladite couche à libération contrôlée jouxtant le substrat et ladite couche supplémentaire étant disposée de façon à ce qu'elle jouxte ladite couche d'adhésif, espacée de ladite couche à libération contrôlée.

16. Dispositif multicouche selon la revendication 14, ladite couche à libération contrôlée se trouvant à l'état sec dans ledit dispositif, de façon à que ladite couche à libération contrôlée soit exempte d'humidité suite à la formation de ladite couche à libération contrôlée à partir de ladite composition à libération contrôlée.

17. Utilisation de la composition à libération contrôlée selon l'une quelconque des revendications 1 à 13 pour réaliser un dispositif multicouche destiné à l'application sur un substrat.

18. Composition à libération contrôlée selon l'une quelconque des revendications 1 à 13, destinée à l'utilisation dans la médecine.
